# EUROPEAN PATENT SPECIFICATION

(11) **EP 4 178 551 B1**
(45) Date of publication and mention of the grant of the patent: **07.08.2024**
(21) Application number: 21758815.1
(22) Date of filing: 08.06.2021
(51) Int. Cl.: A61K 9/48, A61K 31/12, A61K 36/9066, A61K 9/00

(54) **CURCUMINOID COMPOSITIONS WITH HIGH BIOAVAILABILITY**
CURCUMINOIDZUSAMMENSETZUNGEN MIT HOHER BIOVERFÜGBARKEIT
COMPOSITIONS CURCUMINOÏDES À BIODISPONIBILITÉ ÉLEVÉE

(30) Priority: 09.07.2020 WO PCT/TR2020/050612
(43) Date of publication of application: 17.05.2023
(73) Proprietor: Pisak, Mehmet Nevzat, 34337 Besiktas/Istanbul (TR)
(72) Inventor: Pisak, Mehmet Nevzat, 34337 Besiktas/Istanbul (TR)
(74) Representative: Coral, Nükhet Serra Yardimci
(86) International application number: PCT/TR2021/050552
(87) International publication number: WO 2022/010439

(56) References cited:
- EP-A1- 2 229 940
- WO-A1-2010/106191
- WO-A1-2017/196632
- WO-A1-2020/065548
- WO-A2-2009/101263
- WO-A2-2013/132457
- WO-A2-2021/006850
- US-A1- 2015 025 104

## Description

### TECHNICAL FIELD

The present invention relates to a pharmaceutical composition comprising a curcuminoid compound, an emulsifier, an acid component, a dextrin compound and a silica.

### BACKGROUND ART

Turmeric, the natural source of curcumin, has been known to have medicinal properties for thousands of years. Curcumin (1,7-bis(4-hydroxy-3-methoxyphenyl)-1,6-heptadiene-3,5-dione) is the main natural polyphenol found in the rhizome of Curcuma longa (turmeric) and in other Curcuma species. Curcuma longa has been traditionally used in India and Asian countries as a medicinal herb due to, but not limited to, its antioxidant, anti-inflammatory, antimutagenic, antimicrobial, and anticancer properties. Although curcumin is mainly extracted from curcuma longa, it can also be synthetically produced. There are also other curcuminoids, such as demethoxycurcumin (DMC), bisdemethoxycurcumin (BDMC), and tetrahydrocurcumin (THC). A less common curcuminoid; cyclocurcumin, is also naturally found in curcuma longa although in low proportions.

Curcuminoids have found wide use as supplements and functional foods to be used in beverages and food products for general well-being purposes. As mentioned above, because of its medicinal properties, there are multiple ongoing clinical studies for its application as a pharmaceutical product.

However, the effectiveness of curcuma longa extracts in general and particularly its main curcuminoid compound curcumin, as a medicinal product, supplement or functional food/beverage is limited due to its low bioavailability (BA). The mentioned low bioavailability is caused by poor absorption of curcumin due to its low solubility and low permeability. Thus, very high oral doses and repeated dosing must be used to obtain effective blood concentrations. Even so, most of the doses are never absorbed.

There have been many studies in the state of the art to overcome the problem of curcumin solubility and bioavailability, some of which include combinations of curcumin with other extracts such as piperine or curcumin phytosome formulations. Furthermore, special formulation technologies including liposomes and curcumin complexes with emulsifying agents and other compounds such as cyclodextrins are used to achieve the required solubility. The use of cyclodextrins in order to increase the solubility of curcuminoids are studied in the art. The prior art especially focuses on γ-cyclodextrin. Purpura et. al, on their study (Analysis of different innovative formulations of curcumin for improved relative oral bioavailability in human subjects, 2016) discloses a new γ-cyclodextrin and curcumin formulation which shows the highest plasma concentration of curcuminoids. It is also disclosed therein that as γ-cyclodextrin is completely digested by salivary and pancreatic amylase, it is preferred to α- and β-cyclodextrin.

EP 2 249 852 discloses the use of γ-cyclodextrin for providing a water-soluble and stable pharmaceutical composition of curcumin. In the document, it is disclosed that curcumin formulations of this application, even at high concentration of curcumin, are soluble in an alkaline aqueous solution containing γ-cyclodextrin. However, this formulation is mainly developed for parenteral application, which is not a convenient way for the administration of curcumin on a regular basis.

For oral treatments, EP 2 408 442 discloses an oral pharmaceutical, nutraceutical or functional food composition comprising curcumin, citric acid and polysorbate as an emulsifier with high viscosity to provide a stable composition. Likewise, the use of polysorbate in order to increase the solubility and the bioavailability of curcumin is also known from EP 2 934 185.

WO2010/106191 A1 discloses a composition comprising pure curcumin, citric acid and Tween 80 encapsulated in a gelatin capsule. Further WO2010/106191 A1 and EP2 229 940 disclose a tablet preparation comprising curcumin, polysorbate 60, silicon dioxide and citric acid.

However, there is still a need in the art for an oral composition comprising a curcuminoid compound wherein the solubility and bioavailability of the curcuminoid compound is enhanced, and at the same time the composition being stable, easy to manufacture and affordable.

### SUMMARY OF THE INVENTION

The present invention provides an oral composition comprising at least one curcuminoid compound, at least one emulsifier, at least one acid component, at least one dextrin compound and at least one silica.

In one embodiment, the composition of the present invention contains an organic acid as acid component and preferably an acid component which is selected from the group consisting of stearic acid, citric acid, gluconic acid (E574), inosinic acid (E630), glutamic acid (E620), guanilic acid (E626), sodium caprate (decanoic acid), dichroic acid (E330), malic acid (E296), acetic acid (E260), tartaric acid (E334), lactic acid (E270) and alginic acid (E400). As mentioned above, the composition contains at least one acidic component, therefore, any combination of the acids disclosed in the group above can be used in the formulation of the present invention. The composition most preferably comprises stearic acid, citric acid or decanoic acid (sodium caprate).

In another embodiment, the present invention provides a composition comprising a curcuminoid compound, an organic acid, a dextrin compound and an emulsifier having a HLB value of between 10 and 25.

In the present invention, the emulsifier is preferably selected from the group consisting of polyoxethylene derivatives, sorbitan esters, polyethylene glycol derivatives and their combinations thereof. Polyoxethylene can be a polyoxyglyceride such as stearoyl polyoxyl-32 glyceride, lauroyl polyoxyl-32 glyceride; or a polyoxyethylene sucrose diester dimyristate, polyoxyethylene sucrose diester dipalmitate, polyoxyethylene sucrose diester dioleate. Sorbitan esters can be polysorbate 80, polysorbate 60 or polysorbate 20. Polyethylene glycol derivatives can be PEG-8 laurate, PEG 400 monoluarate, PEG 10 isooctylphenyl ether, PEG 40 stearate, PEG 50 stearate, or PEG 40 isooctylphenyl ether.

According to the present invention, the best results are obtained when the weight ratios of the ingredients are in a specific range. In one embodiment, the weight ratio of the emulsifier to the acid component is between 40:1 and 1:1, preferably between 30:1 and 2:1 and most preferably between 20:1 and 5:1. In another embodiment, the weight ratio of the dextrin compound to the emulsifier is between 1:0.5 and 1:15, preferably between 1:1 and 1:10, most preferably between 1:2 and 1:7. In another embodiment, the weight ratio of curcuminoid compound to the dextrin compound is between 0.1:1 and 10:1, preferably 0.5:1 and 8:1, most preferably between 1:1 and 5:1. In another embodiment, the weight ratio of the silica derivative to curcuminoid compound is between 0.5:1 and 10:1, preferably between 1:1 and 8:1, most preferably between 1:1 and 5:1.

### DETAILED DESCRIPTION OF THE INVENTION

The present invention relates to an oral composition comprising at least one curcuminoid compound, at least one emulsifier, at least one silica, at least one dextrin compound and at least one acid component, thereby providing an effective curcuminoid composition.

The composition of the present invention preferably contains an organic acid as acid component or more preferably an acid component which is selected from the group consisting of stearic acid, citric acid, gluconic acid (E574), inosinic acid (E630), glutamic acid (E620), guanilic acid (E626), sodium caprate (decanoic acid), dichroic acid (E330), malic acid (E296), acetic acid (E260), tartaric acid (E334), lactic acid (E270) and alginic acid (E400). The most preferred acid compound is stearic acid.

According to the present invention, the composition comprises at least one acid component in an amount of from 1 to 100 mg, preferably from 5 to 100 mg,more preferably from 5 to 75 mg, most preferably from 5 to 50 mg per oral unit dose.

In a preferred embodiment the weight ratio of the acid component to the emulsifier is between 1:40 and 1:1, preferably between 1:30 and 1:2 and most preferably between 1:20 and 1:5. The curcuminoid compound is preferably selected from the group consisting of bisdemethoxycurcumin, desmethoxycurcumin, dimethylcurcumin, curcumin and their combinations thereof. The most preferred curcuminoid is curcumin.

According to the present invention, the composition comprises curcumin in an amount of from 20 to 400 mg, preferably from 30 to 300 mg, more preferably from 40 to 200 mg, most preferably from 60 to 120 mg per oral unit dose.

In one embodiment, the weight ratio of curcuminoid compound, preferably curcumin, to the dextrin compound is between 0.1:1 and 10:1, preferably between 0.5:1 and 8:1 most preferably between 1:1 and 5:1.

In a preferred embodiment dextrin compound of the present invention is selected from the group consisting of α-cyclodextrin, β-cyclodextrin, 2-hydroxypropyl-β-cyclodextrin, sulfobutylether β-cyclodextrin sodium salt, randomly methylated β-cyclodextrin, branched β-cyclodextrin, γ-Cyclodextrin and their derivatives thereof. The cyclodextrin is preferably selected from the group consisting of β-cyclodextrin, 2-hydroxypropyl-β-cyclodextrin, sulfobutylether β-cyclodextrin sodium salt, randomly methylated β-cyclodextrin, and branched β-cyclodextrin. The cyclodextrin of the present invention is most preferably β-cyclodextrin or maltodextrin.

It has also been found that when the composition according to the present invention comprises a β-cyclodextrin, the solubility of curcuminoid compound becomes much better compared to the compositions comprising γ-cyclodextrin as a dextrin compound. In addition, compositions of the present invention provide higher levels of solubility at much lower amounts of cyclodextrin compared to the state of the art.

In one embodiment of the present invention, the composition comprises at least one dextrin compound in an amount of from 25 to 400 mg, preferably from 30 to 400 mg, more preferably from 30 to 300 mg per oral unit dose.

In another embodiment, the weight ratio of the dextrin compound to the emulsifier is between 1:0.5 and 1:15, preferably between 1:1 and 1:10, most preferably between 1:2 and 1:5.

The emulsifier of the present invention preferably has an HLB value of between 10 and 25, more preferably between 10 and 21.

As used herein, HLB means hydrophilic-lipophilic balance (HLB), i.e. the balance of the size and strength of the hydrophilic (water-loving or polar) and the lipophilic (oil-loving or non-polar) groups of the emulsifier. In the HLB system, each emulsifier is assigned a numerical value which is called its HLB. The HLB of emulsifiers is shown in all current ICI emulsifier literature, and similar values may be calculated or estimated by various means for any emulsifier. All emulsifiers consist of a molecule that combines both hydrophilic and lipophilic groups. An emulsifier that is lipophilic in character is assigned a low HLB number (below 9.0), and one that is hydrophilic is assigned a high HLB number (above 10.0). Those in the range of 9-11 are intermediate.

In one embodiment the emulsifier is selected from the group consisting of polyoxethylene derivatives, sorbitan esters, polyethylene glycol derivatives and their combinations thereof. Polyoxethylene can be polyoxyglycerides such as stearoyl polyoxyl-32 glycerides, lauroyl polyoxyl-32 glycerides; or a polyoxyethylene sucrose diesterdimyristate, polyoxyethylene sucrose diesterdipalmitate, polyoxyethylene sucrose diesterdioleate. Sorbitan esters can be polysorbate 80, polysorbate 60 or polysorbate 20. Polyethylene glycol derivatives can be PEG-8 laurate, PEG 400 monoluarate, PEG 10 isooctylphenyl ether, PEG 40 stearate, PEG 50 stearate or PEG 40 isooctylphenyl ether.

In another embodiment emulsifier is a polyoxylglyceride or polysorbate; preferably a polysorbate. These polyoxylglycerides are obtained by reacting glycerides with polyoxyethylene glycols, and as a result, they contain mixtures of monoesters and diesters of PEG with long- and medium-chain fatty acids along with monoglycerides, diglycerides and triglycerides of fatty acids. Because of their amphiphilic properties due to the presence of relatively long-chain hydrophilic PEG esterified with long hydrophobic chain fatty acids, polyoxylglycerides are surface-active agents having different hydrophilic-lipophilic balance (HLB) depending on the chain length of PEG and the nature of fatty acids present. Thus, the emulsifier used in the present composition is preferably stearoyl polyoxyl-32 glyceride (Acconon C-50/ Gelucire 50/13) or lauroyl polyoxyl-32 glyceride (Acconon C-44/ Gelucire 44/14).

Although substantially higher levels of solubility compared to marketed products containing either only an emulsifier or only a cyclodextrin compound can be achieved with the composition of the present invention comprising a cyclodextrin compound and an emulsifier such as a sorbitan ester (polysorbate 80); it has also been surprisingly discovered that polyoxyethylene derivatives and preferably polyoxylglycerides can have an even stronger effect on the solubility of curcumin when combined with a cyclodextrin. The amphiphilic nature of polyoxylglycerides (polyoxyethylene derivatives) combined with their high HLB value, coupled with the hydrophilic nature of the cyclodextrins results in this synergistic composition with a highly soluble and bioavailable curcumin complex. It has also been discovered that silica derivatives, preferably ones with a high surface and tamped density improves the solubility and bioavailability of curcumin.

According to the present invention, the composition comprises at least one emulsifier in an amount of between 10% and 90%, preferably between 10% and 80% and more preferably between 20% and 60% by the total weight of the composition.

Dextrins are a group of low-molecular-weight carbohydrates produced by the hydrolysis of starch or glycogen. One preferred type of dextrin of the present invention is maltodextrin and the other is a cyclodextrin.

Maltodextrin is a short-chain starch sugar used as a food additive in prior art. It is produced also by enzymatic hydrolysis from gelled starch and is usually found as a creamy-white hygroscopic spray-dried powder. Maltodextrin is easily digestible, being absorbed as rapidly as glucose, and might either be moderately sweet or have hardly any flavor at all.

The cyclical dextrins are known as cyclodextrins. They are formed by enzymatic degradation of starch by certain bacteria, for example, Paenibacillus macerans (Bacillus macerans). Cyclodextrins have toroidal structures formed by 6-8 glucose residues.

The preferred dextrin compounds of the present invention are selected from the group consisting of beta cyclodextrins and their derivatives including: β-cyclodextrin, 2-hydroxypropyl-β-cyclodextrin, sulfobutylether β-cyclodextrin sodium salt, randomly methylated β-cyclodextrin, branched β-cyclodextrin and maltodextrin.

Although y-cyclodextrin compounds can also be employed, the preferred embodiment of the invention entails the use of beta cyclodextrins including beta cyclodextrin (BCD), DM-β-cyclodextrin, RM-β-cyclodextrin and hydroxypropyl β-cyclodextrin (HPBCD), which have enhanced the solubility of curcuminoid even with the least expensive cyclodextrin compound β-cyclodextrin. In addition, formulations in the prior art have focused on extremely high ratios of cyclodextrin compounds such as HPBCD, contrary to the findings of the present invention that provides significantly higher levels of solubility with lower amounts of cyclodextrin compared to state of the art, when combined with emulsifiers that have a high HLB value.

Although newer and significantly more expensive dextrin compounds such as DM-β-cyclodextrin and RM-β-cyclodextrin can also be employed within the compositions of the present invention, it has been found that by the formulation of the present invention the solubility of curcuminoid compound is enhanced with the least expensive dextrin compound, i.e. β-cyclodextrin. β-cyclodextrin provides a better solubility compared to the compositions found in the prior art which have employed methylated dextrin compounds.

Although substantially higher levels of solubility compared to prior art containing either only an emulsifier or only a dextrin compound can be achieved with the composition comprising a dextrin compound and an emulsifier; it has also been discovered that polyoxyethylene derivatives and preferably sorbitan esters can have an even stronger effect on the solubility of curcuminoid compounds when combined with a cyclodextrin. The amphiphilic nature of sorbitan esters, combined with their high HLB value, coupled with the hydrophilic nature of the dextrins, further combined with a silica derivative (preferably hydrophilic) results in this synergistic composition with a highly soluble and bioavailable curcuminoid inclusion complex that is also stable.

Silica derivatives have been used in oral dosage formulations for decades, there are many different silica derivatives used for various applications (i.e: to increase flowability, compressibility etc.)

The preferred silica derivatives of the present disclosure have an extremely low bulk density and high surface area. These silica derivatives have a mean particle diameter of 10 to 250 micron (determined according to the laser diffraction method) and a BET surface area of 40 to 400 m²/g (determined according to DIN 66 131 with nitrogen). The silica derivatives also typically have a pore volume of about 0.5 to 2.5 mL/g, wherein less than about 5% of the overall pore volume has a pore diameter of less than about 5 nm, the remainder being mesopores and macropores. Additionally, the silica derivatives typically have a pH in the range of about 3.4 to about 8, preferably have a tamped (tapped) density of about 50 to 600 g/L and most preferably a tamped density of between 50 and 400 g/L and they are most preferably hydrophilic (The tapped density is calculated according to ISO 787-11 and converted to the value in g/L).

As used herein, BET surface area means the surface area of a solid in relation to its mass, measured in m²/g. As defined in DIN 66131, it is generally measured based on the BET method (Brunauer, Emmett, Teller, in Journal of the American Chemical Society 60 (1938), p. 309). As used herein, tamped (tapped) density means a measured variable that describes the amount of volume lost by a powdered solid when it is shaken or packed down firmly as defined by ISO 787-11.

The silica of the present invention is preferably calcium silicate (such as Zeopharm), most preferably zeopharm 5170; or magnesium aluminometasilicate (such as Neusillin), most preferably Neusillin US2; or colloidal silicon dioxide, most preferably AEROPERL^{®} 300. The specific silica material that was used in the studies of the invention for compositions and methods is AEROPERL^{®} 300 (a hydrophilic silica derivative), which is available from Evonik Degussa AG, Dusseldorf, Germany. However, other silica derivatives that have similar physical and chemical properties described herein can also be used.

In one embodiment of the disclosure, the particles of the silica derivative have a mean grain (particle) diameter of 10-120 micron. According to the present invention, particles of silica derivatives have a BET surface area of at least 150 m²/g, 200 m²/g, 250 m²/g or 275 m²/g.

It was surprisingly found in the experiments of the present invention that the addition of one such silica (Aeroperl 300) provides significantly higher levels of solubility compared to curcumin only combined with a dextrin, an emulsifier and an organic acid.

Most emulsifiers suitable for use in the present invention are in liquid or semi liquid form and this creates a problem to be solved in order to manufacture solid curcuminoid compositions. If the compositions of the present disclosure need to be formulated in solid form when employing a liquid or semi liquid emulsifier such as Tween 80, silica derivatives are the preferred excipient(s). Silica derivatives have been used in oral dosage formulations for decades, there are many different silica derivatives used for various applications (i.e: to increase flowability, compressibility etc.) In the present invention, it has been discovered that the use of silica does not have any negative impact and can even have a positive effect on the biovailability of curcuminoids.

According to the present invention, the oral composition formulated with the composition of the present invention can be solid, semi-solid or liquid form. Another advantage of the present invention is that the compositions can be in solid form (powder form). In the prior art, solid form of curcuminoid formulation has significant limitation on the solubility and bioavailability therefore its products on the market, especially ones claiming the highest bioavailability and solubility, are generally in oil or liquid form. Although higher amounts of an emulsifier can be employed within the composition of the present invention, the use of high amounts (higher than 75% by volume) of high HLB value emulsifiers is not preferable because it has been found that emulsifiers can impair the function of the mucosal barrier and increases the permeability of the gut especially at high doses. Although this would increase the permeability of a curcuminoid from the intestinal mucosal barrier, it will also damage the mucosal barrier itself, especially with long term use and lead to other secondary complications.

According to the present invention, the composition comprises curcuminoid compound in an amount of from 5% to 60%, preferably from 5% to 50%, more preferably from 5% to 40% and most preferably from 5% to 30% by the weight of the composition.

According to the present invention, the composition comprises at least one emulsifier in an amount of from 20 to 90%, preferably from 30 to 90% and more preferably from 40 to 90% by the weight of the composition.

According to the present invention, the composition comprises at least one silica in an amount of from 1% to 70%, preferably from 10% to 60% and more preferably from 20% to 60% by the weight of the composition.

According to the present invention, the composition comprises at least one dextrin compound in an amount of from 1% to 50%, preferably from 2% to 25% and more preferably from 2% to 15% by the weight of the composition.

For the avoidance of doubt, the term "weight of the composition" used in the calculations means the sum of the weights of the curcuminoid compound, emulsifier, acid component, dextrin compound and silica derivative. Moreover, it has been found that the stability of the composition of the present invention is excellent at room temperatures (25°C ± 2°C/40% RH ± 5%) with air-tight packaging, up to a period of 6 months, wherein the curcumin content of the composition is not less than 90% compared with day "0".

In one embodiment, the curcuminoid compound has at least 50% of curcumin content, preferably at least 55%, more preferably at least 65%, and most preferably at least 70% of curcumin content.

For the avoidance of doubt; the term "per oral unit dose" means: the weight of the curcuminoid compound or the weight of the dextrin compound or the weight of the silica derivative or the weight of the emulsifier within a single administration, such as a single tablet, a single capsule, or the amount of drops that would be described in a patient information leaflet as the amount of the curcuminoid that would be taken by the patient (e.g; 5 puffs of an oral spray or 20 drops of a solution) taken at a single time. As for food and beverages, the single unit dose would be equal to the serving size of a beverage or food product (e.g; if a curcuminoid containing cake is 500 grams but a single serving is written on the label as 100 grams, the single unit dose would be 100 grams) The same principle would be applied to beverages.

In a preferred embodiment the oral unit dosage forms prepared with the composition of the present invention is administered 1 to 4 times daily and usually does not need to be administered more than 6 times due to the superior solubility and significant bioavailability that can be attained.

The oral dosage forms prepared with the compositions of the present invention can be used for the treatment or prophylaxis of a variety of diseases and/or medical conditions. The mentioned diseases include, but are not limited to, Acquired Hypothyroidism, Acute Gastritis, Acute Pain, Agoraphobia, AIDS Related Illness, Alcohol Abuse, Alcoholism, Alopecia Areata, Alzheimer's Disease, Amphetamine Dependency, Amyloidosis, Amyotrophic Lateral Sclerosis (ALS), Angina Pectoris, Ankylosis, Anorexia, Anorexia Nervosa, Anxiety Disorders, any chronic medical symptom that limits major life activities, any Chronic Medical Symptom that Limits Major Life Activities, Arteriosclerotic Heart Disease, Arthritis, Arthritis (Rheumatoid), Arthropathy, gout, Asthma, Attention Deficit Hyperactivity Disorder (ADD/ADHD), Autism/Asperger's, Autoimmune Disease, Back Pain, Back Sprain, Bell's Palsy, Bipolar Disorder, Brain Tumor, Breakthrough Pain, Malignant, Bruxism, Bulimia, Cachexia, Cancer, Carpal Tunnel Syndrome, Cerebral Palsy, Cervical Disk Disease, Cervicobrachial Syndrome, Chemotherapy Chronic Fatigue Syndrome, Chronic Pain, Chronic renal failure, Cocaine Dependence, Colitis, Conjunctivitis, Constipation, Crohn's Disease, Cystic Fibrosis, Damage to Spinal Cord Nervous Tissue, Darier's Disease, Degenerative Arthritis, Degenerative Arthropathy, Delirium Tremens, Dermatomyositis, Diabetes, Diabetic Neuropathy, Diabetic Peripheral Vascular Disease, Diarrhea, Diverticulitis, Dysthymic Disorder, Eczema, Emphysema, Emphysema, Endometriosis, Epidermolysis Bullosa, Epididymitis, Epilepsy, Felty's Syndrome, Fibromyalgia, Friedreich's Ataxia, Gastritis, Genital Herpes, Glaucoma, Glioblastoma Multiforme, Graves Disease, Cluster Headaches, Migraine Headaches, Tension Headaches, Hemophilia A, Henoch-Schonlein Purpura, Hepatitis C, Hereditary Spinal Ataxia, HIV/AIDS, Hospice Patients, Huntington's Disease, Hypertension, Hypertension, Hyperventilation, Hypoglycemia, Impotence, Inflammatory autoimmune-mediated arthritis, Inflammatory Bowel Disease (IBD), Insomnia, Intermittent Explosive Disorder (IED), Intractable Pain, Intractable Vomiting, Lipomatosis, Lou Gehrig's Disease, Lyme Disease, Lymphoma, Major Depression" General Anxiety Disorder, Malignant Melanoma, Mania, Melorheostosis, Meniere's Disease, Motion Sickness, Mucopolysaccharidosis (MPS), Multiple Sclerosis (MS), Muscle Spasms, Muscular Dystrophy, Myeloid Leukemia, Nail-Patella Syndrome, Nightmares, Obesity, Obsessive Compulsive Disorder, Opiate Dependence, Osteoarthritis, Panic Disorder, Parkinson's Disease, Peripheral Neuropathy, Peritoneal Pain, Persistent Insomnia, Porphyria, Post Polio Syndrome (PPS), Post-traumatic arthritis, Post-Traumatic Stress Disorder (PTSD), Premenstrual Syndrome (PMS), Prostatitis, Psoriasis, Pulmonary Fibrosis, Quadriplegia, Radiation Therapy, Raynaud's Disease, Reiter's Syndrome, Restless Legs Syndrome (RLS), Rheumatoid Arthritis, Rheumatoid Arthritis, Rheumatoid Arthritis, Rosacea, Schizoaffective Disorder, Schizophrenia, Scoliosis, Sedative Dependence, Seizures, Senile Dementia, Severe Nausea, Shingles (Herpes Zoster), Sinusitis, Skeletal Muscular Spasticity, Sleep Apnea, Sleep Disorders, Spasticity, Spinal Stenosis, Sturge-Weber Syndrome (SWS), Stuttering, Tardive Dyskinesia (TD), Temporomandibular joint disorder (TMJ), Tenosynovitis, Terminal Illness, Thyroiditis, Tic Douloureux, Tietze's Syndrome, Tinnitus, Tobacco Dependence, Tourette's Syndrome, Trichotillomania, Viral Hepatitis, Wasting Syndrome, Whiplash, Wittmaack-Ekbom's Syndrome, Writers' Cramp, nausea, vomiting, premenstrual syndrome, unintentional weight loss, insomnia, and lack of appetite, spasticity, painful conditions, especially neurogenic pain, movement disorders, asthma, glaucoma, adrenal disease, inflammatory bowel disease, migraines, fibromyalgia, and related conditions, multiple sclerosis, spinal cord injuries. It exhibits antispasmodic and muscle-relaxant properties as well as stimulates appetite. Other studies state that cannabis or curcuminoids may be useful in treating alcohol abuse, amyotrophic lateral sclerosis, collagen-induced arthritis, asthma, atherosclerosis, bipolar disorder, colorectal cancer, HIV-Associated Sensory Neuropathy, depression, dystonia, epilepsy, digestive diseases, gliomas, hepatitis C, Huntington's disease, leukemia, skin tumors, methicillin-resistant Staphylococcus aureus (MRSA), Parkinson's disease, pruritus, posttraumatic stress disorder (PTSD), psoriasis, sickle-cell disease, sleep apnea, and anorexia nervosa, drug dependence, moderate to severe pain management, insomnia, anxiety, epilepsy, seizures, parkinson's and also for the treatment of various cancers including blood cancers and solid tumors.

In another embodiment of the present invention, the use of the composition in oral dosage forms, further comprises at least one other acceptable excipient as a carrier.

Oral dosage forms of the present invention may comprise other excipients such as: suitable diluents, binders, lubricants, preservatives, antioxidants, flavoring agents, disintegrating agents, surfactants, glidants, sweetening agents, coloring agents and coating agents as pharmaceutically acceptable excipients and preferably disintegrant, lubricant or mixtures thereof.

Moreover, due to the composition of this invention, quality, such as the external appearance, bitter taste, aftertaste, is improved, even without the addition of extra sweeteners and flavoring agents. This aspect of the invention is especially important for manufacturing food and beverages with curcuminoid content.

Oral dosage forms of the present invention may be tablet, capsule, gel capsule, pellet, granule, hard gelatine capsule, sachet in powder, liquid, tablet in tablet, tablet in capsule, powder or coated tablet, preferably being tablet or capsule. The oral dosage forms may also be in the form of drop, pellet, granule, solution, suspension, syrup, powder, preferably made for sublingual or oromucosal application in the form of drops, sublingual tablets, sprays, strips or as a chewing gum.

Acceptable diluents of the present invention may be selected from the group consisting of magnesium stearate, lactose, microcrystalline cellulose, starch, pre-gelatinized starch, calcium phosphate, calcium sulphate, calcium carbonate, sodium starch glycolate, mannitol, sorbitol, xylitol, sucrose, maltose, fructose, dextrose and the like.

Acceptable binders of the invention may be selected from the group consisting of starches, natural sugars, corn sweeteners, natural and synthetic gums, cellulose derivatives, gelatin, polyvinylpyrrolidone, polyethylene glycol, waxes, sodium alginate, alcohols, water and the like.

Acceptable lubricants of the present invention may be selected from the group consisting of metallic stearates, metallic lauryl sulfates, fatty acids, fatty acid esters, fatty alcohols, paraffins, hydrogenated vegetable oils, polyethylene glycols, boric acid, polyvinylpyrrolidone, sodium benzoate, sodium acetate, sodium chloride, talk and the like.

Acceptable disintegrating agents of the present invention may be selected from the group consisting of starches, cellulose derivatives, polyvinylpyrrolidone, crospovidone, clays, ionexchange resins, alginic acid, sodium alginate and the like.

Flavoring agent(s) that may be used in the invention is meant to impart a pleasant flavor and/or odor to the oral composition. Suitable flavoring agents include but not limited to natural and artificial flavors, such as synthetic flavor oils and flavoring aromatics and/or natural oils, extracts from plants, leaves, flowers, fruits and so forth and combinations thereof. Representative suitable flavoring agents may be for example, without limitation, menthol, cinnamon, wintergreen, clove, bay, anise, eucalyptus, thyme, cedar leave, nutmeg, sage, bitter almonds and cassia, vanilla, artificial vanilla, chocolate, artificial chocolate, bubble gum, both natural and artificial fruit flavors, such as cherry flavor, grape flavor, orange flavor, banana flavor, strawberry flavor, lemon flavor, grapefruit flavor and "mint" flavors such as peppermint flavor and spearmint flavor, lime flavor, apple flavor, pear flavor, peach flavor, raspberry flavor, plum flavor, pineapple flavor, apricot flavor and so forth, including combinations of two or more thereof. Flavoring agents are generally provided as a minor component of the composition in amounts effective to provide a palatable flavor to the composition. The amount of flavoring agent may depend on a number of factors, including the desired organoleptic effect. The precise amount of sweetening and/or flavoring agent(s) depends on the properties of the agent(s) used, however generally in an amount that is sufficient to mask the unpleasant taste and/or odor associated with curcuminoids as determinable by one skilled in the art. However, flavoring agents generally present is in a pharmaceutically or nutraceutically acceptable range.

Sweeteners suitable for inclusion in the present invention may be determined by one skilled in the art including, for example without limitation, both natural and artificial sweeteners such as the representative sweetening agents of intense sweeteners such as sorbitol, sucrose, saccharine such as sodium saccharin, cyclamates such as sodium cyclamates, aspartame, sucralose, thaumatin, acesulfam K, and the like, and sugars such as monosaccharides, disaccharides and polysaccharides. Representative sugars useful in the present invention include, without limitation, xylose, ribose, glucose, mannose, galactose, fructose, dextrose, sucrose, maltose, partially hydrolyzed starch or corn syrup, and sugar alcohols such as sorbitol, xylitol, mannitol, glycerin, etc. and combination thereof. Presently preferred as a sugar sweetener is sucralose. Sugar sweeteners may be replaced or augmented by water-soluble artificial sweeteners, such as the suitable artificial sweeteners previously listed and mixtures thereof. The amount of artificial sweetener used in the composition may vary to provide an appropriate amount of sweetness as determinable by one skilled in the art. Mixtures of sweetening and/or flavoring agents are preferably used.

Examples of preservatives suitable for use in the present invention include, for example without limitation, one or more alkyl hydroxybenzoates, such as methyl hydroxybenzoates, ethyl hydroxybenzoates, propyl hydroxybenzoates, butyl hydroxybenzoates and the like. Additional preservatives useful in the present invention include, but are not limited to, sodium benzoate, potassium sorbate, salts of edetate (also known as salts of ethylenediaminetetraacetic acid, or EDTA, such as disodium edetate), pimaricin based preservatives and antimicrobial agents including parabens (p-hydroxybenzoic acids esters) such as methyl paraben, ethylparaben, propylparaben, butylparaben and the like, and combinations thereof. although other pharmaceutically acceptable preservatives may be substituted, therefore. Preservative(s) as used in the composition are in an acceptable range.

The composition may also contain a viscosity enhancing agent(s) which include but are not limited to gums; sorbitol; glycerol; polyvinyl alcohol; polyvinyl pyrrolidone; polyethylene oxide; cellulose derivatives, such as hydroxypropyl methylcellulose or a salt thereof, alkyl ether of cellulose, such as methylcellulose, ethyl cellulose, hydroxyethyl cellulose, hydroxyethyl methylcellulose and mixtures thereof. Preferably the viscosity-enhancing agent is hydroxypropyl methylcellulose e.g. (HPMC K4M, HPMC K100 LVP; HPMC K15 MP; HPMC E4 MP; HPMC E10 MP CR).

The composition may also contain a pH-stabilizing agent to maintain a desired pH. The term "pH-stabilizing agent" encompasses buffers and pH-altering agents. Suitable pH-stabilizing agents include but not limited to tribasic sodium phosphate, anhydrous sodium carbonate, glycine, citric acid or mixtures thereof.

Preferably the pH of the composition is in a range of about 3 to about 7.5.

Most preferably the pH of the composition is in a range from about 5 to about 7.5.

The composition may also contain antioxidant(s) preferably selected as tocopherols, gallic acid and gallates, isomaltulose, butylated hydroxy anisole, butylated hydroxy toluene, ascorbic acid, maleic acid, sodium bisulphate, sodium metabisulphite or sodium formaldehyde sulphoxylate.

In a preferred embodiment, when the composition of the present invention is used in a unit dose formulation, the pH-stabilizing agent(s), antioxidant(s) ,preservative(s) may be included in the manufacturing of the finished dosage formulation to be used by the end consumer/patient, to increase shelf life of the final product; they may be included from about 0.001% to about 20% of the tablet, capsule, beverage or food product based on the total weight of a single unit dose or single serving size.

The most preferred emulsifiers of the present invention such as polysorbate 80, polysorbate 60, polysorbate 20 are all available both as nutraceutically and pharmaceutically acceptable excipients providing flexibility for commercializing/registering the compositions of the present invention. The same advantage is also attained with most dextrins, especially beta cyclodextrin which is also pharmaceutically and nutraceutically acceptable excipients. And silica derivatives, such as colloidal silicon dioxide are also a pharmaceutically and nutraceutically acceptable excipient. Therefore, the composition of the present invention is developed for use as a medicinal product and/or supplement(s) and/or as beverages or food products.

In another embodiment, the present invention provides oral dosage forms that can be prepared with the composition such as a nutraceutical composition, pharmaceutical composition, beverages or food products (such as nutraceutical bars, cakes, chocolate bars, cookies, ice cream, cereals).

It has also been observed by the inventor that the particle size of the curcuminoid compound has a significant effect on the transparency of the liquid when the composition of the present invention is mixed with water or used in beverages, thus in a preferred embodiment; if the composition of the present invention is to be used in the manufacturing of liquid dosage forms, at least 40% of the particles will have a particle size between 5 and 100 microns and most preferably between 5 and 20 microns as this creates a clearer liquid solution. The particle size of the curcuminoid compound is calculated with the Malvern Mastersizer 3000E. The desired particle size is attained through micronisation. The micronisation can also be done prior to mixing the curcuminoid compound with the excipients according to the present invention to decrease the total weight of the composition to be micronized. Which would be economically more feasible.

In another embodiment, the present disclosure provides a manufacturing method to obtain the oral composition comprising the step of mixing a curcuminoid compound, at least one emulsifier, and at least one acid component, for at least 10 minutes.

In another embodiment, the present disclosure provides a manufacturing method to obtain the oral composition comprising the step of mixing a curcuminoid compound, at least one emulsifier, at least one acid component, at least one dextrin compound and at least one silica derivative for at least 10 minutes.

The method according to the present disclosure comprises the step of mixing firstly the curcuminoid compound with the emulsifier and/or dextrin compound, before mixing with the organic acid or silica derivative.

The mixing can also be performed using a conventional cubic mixer, tumbler, fluid bed dryer or spray dryer depending on the process and the form of excipients employed. The mixing is preferably performed with a high sheer mixer or fluid bed dryer (especially if agglomeration is a desired outcome) The high sheer mixer or fluid bed dryer can also be equipped with a spray nozzle.

The method according to the present disclosure preferably comprises the steps of mixing firstly at least one curcuminoid compound with at least one emulsifier, and then mixing with at least one dextrin compound and with at least one silica derivative.

In a preferred embodiment the method according to the present disclosure comprises the steps of:
a. Mixing the curcuminoid compound and emulsifier under room temperature with a high shear mixer,
b. Adding the dextrin compound and the organic acid,
c. Mixing the mixture of step b) with the silica derivative until the mixture turns into powder form,
d. Sieving the powder mixture through a 200 µm to 2000 µm sieve.

The method according to the present disclosure most preferably comprises the steps of:
1. Mixing the curcuminoid compound and emulsifier under room temperature with high sheer mixer at 300 rpm for 25 minutes,
2. Adding the dextrin compound and organic acid and continue mixing for 10 minutes at 300 rpm,
3. Adding the silica derivative after bringing the mixer's speed down to 50 rpm until the mixture turns into powder form,
4. Sieve the powder mixture from step 3 with a 500 µm stainless steel sieve.

Unless otherwise defined, all technical and scientific terms used herein have the same meaning as commonly understood by one of ordinary skill in the art.

The invention now will be described in particularity with the following illustrative examples.

### EXAMPLES

### MANUFACTURING EXAMPLE

The desired dose of the curcuminoid compound which is based on the curcumin content of the compound can vary depending on the extract or synthetic curcumin. For the purposes of obtaining accurate results about the effects of the compositions on the bioavailability of curcumin, a curcuma longa rhizome extract with about 77% net curcumin content was used.

### Example 1

| **Ingredients** | **Amount in Kg** | **Net Curcumin content in Kg** |
|---|---|---|
| Curcuma longa extract | 11,4 | 8,78 |
| Maltodextrin | 5,6 | |
| Stearic Acid | 2,8 | |
| Tween 80 | 21 | |
| Aeroperl | 14 | |

### Manufacturing Process

1. 11, 4 kg of curcuma longa extract and 21 kg of Tween 80 is weighed and mixed in a high sheer mixer at 300 rpm for 25 minutes.
2. 5,6 kg of maltodextrin and 2,8 kg of stearic acid is weighed and added into the mixture described in step 1 and mixed for 15 minutes.
3. 14 kg of Aeroperl 300 is slowly added in 20-30 minutes (to prevent clumps) to the mixture in step 2, whilst mixing at 50 rpm.
4. The resulting powder from step 3 is sieved by 500 um stainless steel sieve.

### Manufacturing Examples for Comparison Purposes

### Example 2 - Reference example

| **Ingredients** | **Amount in Kg** | **Net Curcumin content in Kg** |
|---|---|---|
| Curcuma longa extract | 11,4 | 8,78 |
| Maltodextrin | 5,6 | |
| Tween 80 | 21 | |
| Aeroperl | 14 | |

### Example 3 - Reference example

| **Ingredients** | **Amount in Kg** | **Net Curcumin content in Kg** |
|---|---|---|
| Curcuma longa extract | 11,4 | 8,78 |
| Aeroperl | 5,6 | |
| Tween 80 | 21 | |

### Example 4 - Reference example

| **Ingredients** | **Amount in Kg** | **Net Curcumin content in Kg** |
|---|---|---|
| Curcuma longa extract | 11,4 | 8,78 |
| Stearic Acid | 2,8 | |
| Tween 80 | 21 | |

### Example 5 - Reference example

| **Ingredients** | **Amount in Kg** | **Net Curcumin content in Kg** |
|---|---|---|
| Curcuma longa extract | 11,4 | 8,78 |
| Maltodextrin | 5,6 | |
| Tween 80 | 21 | |

### Animal Bioavailability Study

### MATERIALS AND METHODS

### Animals

48 male and female Wistar rats (aged 10-12 weeks and 240-260 g) 6 in each group, were maintained in an air-conditioned animal's quarter at a temperature of 22 ± 2 °C and a relative humidity of 50 ± 10 %. Food and water were allowed ad libitum. The animals were acclimatized to the facilities for five days, and then fasted with free access to water for 12 h prior to the experiment. All these animals were housed under similar conditions.

### Drug Administration

Bioavailability and pharmacokinetics of curcumin were studied in all the normal state of rats following an oral administration (gavage) according to the table below. Each group were administered an application volume equal to 87,8 mg of human dose (based on a 65kg human weight and 250 g median rat weight). Blood (0.1 ml) was taken from the tail vein prior to administration of test substances (0 h) and after 0.083, 0.167, 0.25, 0.5, 1, 2, 3, 4 and 8h.

Manufacturing Examples 2-5 are Reference Examples 2-5.

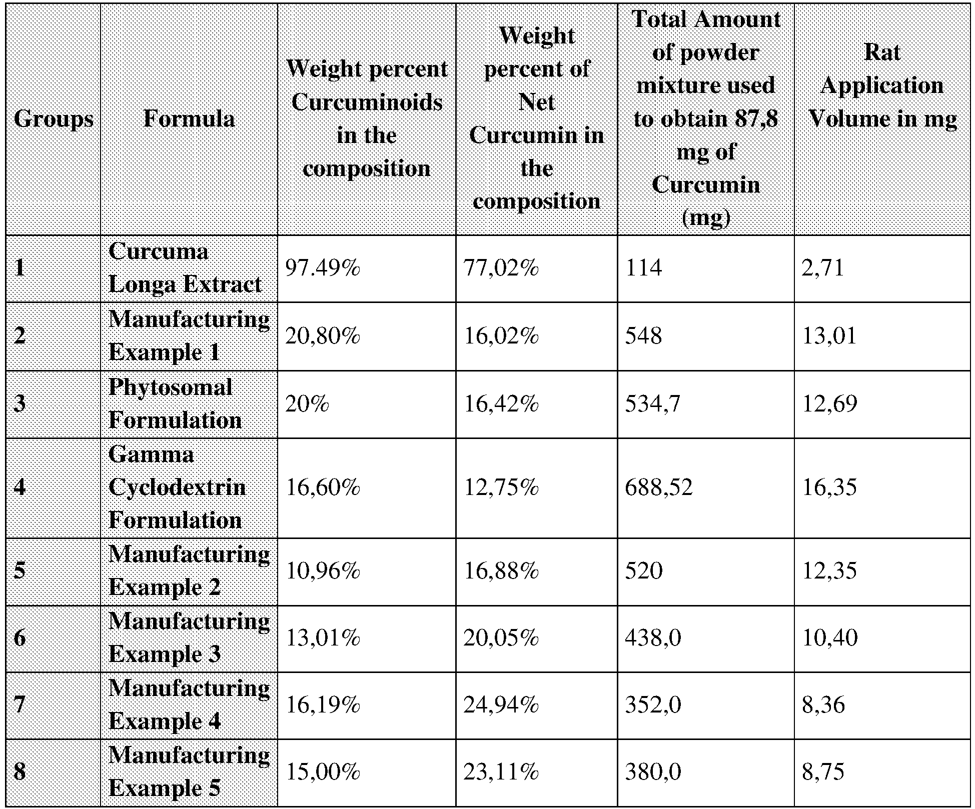

### Extraction of Blood Samples

Blood samples were collected (Parasuman et. al., 2010) in coming tubes and kept on ice until 50 ul dichloromethane (Pargal et al., 1988) was added and they were centrifuged at 7000 x g for 5 min at 4°C and supernatants were collected for HPLC analysis.

### HPLC ANALYSIS

### Chromatographic System :

*Equipment:* High Pressure Liquid chromatography (HPLC); Shimadzu LC-2040 3D Nexera-i
*Column:* 250 mm* 4,6 mm; 5 µm C18 (Inertsil-ODS 3V)
*Flow Rate:* 1.8 ml/min
*Column Temperature:* 35 °C
*Autosampler Temperature:* 4 °C
*Wavelength:* 425 nm
*Injection Volume:* 20 µl
*Run Time:* 7 min
*Diluent:* Methanol
*Mobile Phase:* 5% Acetic acid: Acetonitrile ( 45:55 h/h)
*Buffer pH:* 2,5: 0.01 M H₃PO₄ + 0.01 M NaH₂PO₄.H₂O if necessary, adjust to pH: 2,5 ± 0.2
*Retention Time:* Curcumin ≈ 5.7min

### Pharmacokinetic Results for Different Curcumin Formulations

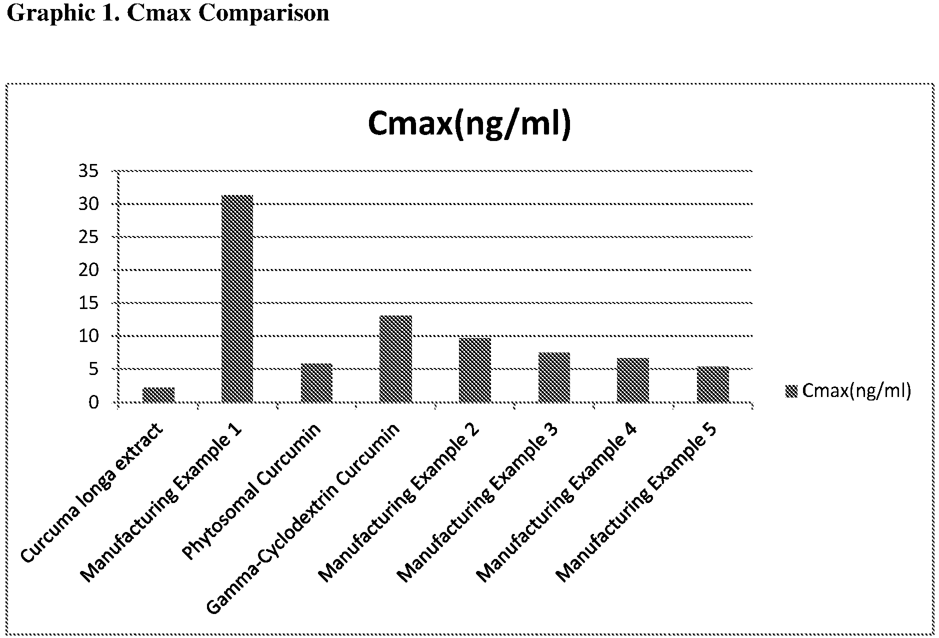

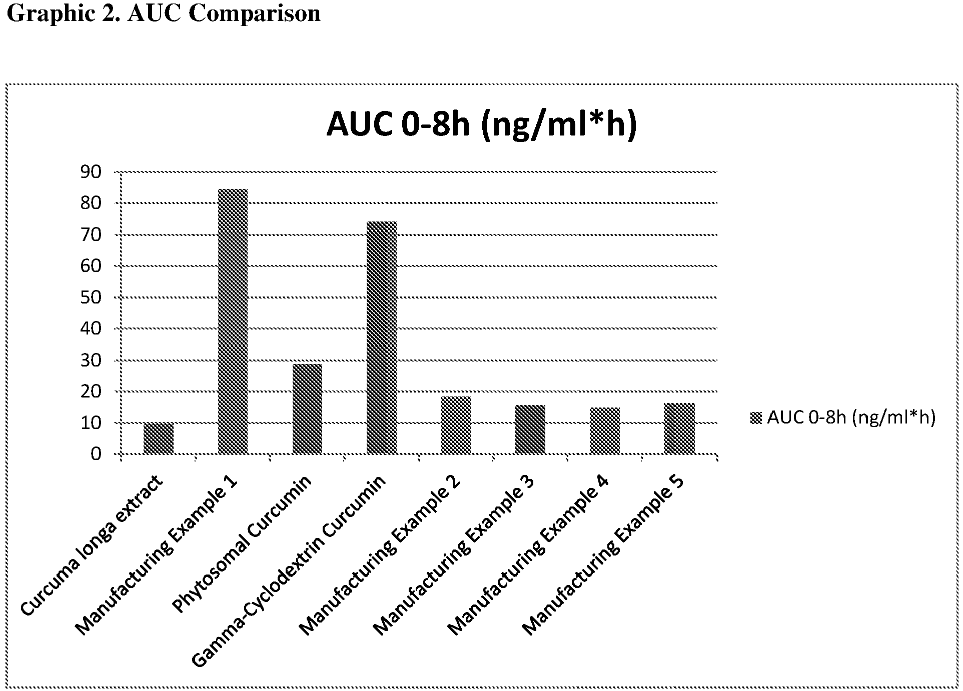

| **PharmacoKinetic Parameters** | **Cmax (ng/ml)** | **Tmax (h)** | **AUC 0-8h (ng/ml*h)** |
|---|---|---|---|
| **Curcuma longa extract** | 2,199 | 0,5 h | 9,606 |
| **Manufacturing Example 1** | 31,322 | 1 h | 84,440 |
| **Phytosomal Curcumin** | 5,794 | 8h | 28,711 |
| **Gamma-Cyclodextrin Curcumin** | 13,053 | 4 h | 74,058 |
| **Manufacturing Example 2** | 9,629 | 0,167 h | 18,340 |
| **Manufacturing Example 3** | 7,443 | 0,5 h | 15.604 |
| **Manufacturing Example 4** | 6,614 | 0,5 h | 14,762 |
| **Manufacturing Example 5** | 5,324 | 1 h | 16.325 |

### Evaluation of the results

The highly synergistic effect of the composition of the present invention is demonstrated by the fact that the composition consisting only of curcumin has provided only about 2,199 ng/ml Cmax, the phytosomal curcumin has provided only about 5,794 ng/ml Cmax, and the gamma cyclodextrin based curcumin provided only about 13,053 ng/ml Cmax, whereas the composition prepared according to the formulation of the present invention, provided 31,322 ng/ml Cmax with a significant Cmax and AUC difference from the other currently marketed formulations. Furthermore, the synergistic effect provided by the composition of the present invention is also evident from the significant difference between manufacturing example 1 and manufacturing examples:2-3-4-5. Thus, demonstrating a longer and stronger effect from the same dose of curcumin based on the pharmacokinetic profile of each respective formulation.

Some examples of oral dosage formulations of curcumin compositions where the compositions of the present invention can be used:

### Example 6

| **Nutraceutical or Pharmaceutical Capsule of Curcumin** | |
|---|---|
| **Ingredients** | **mg** |
| Curcuma longa extract * | 114 |
| Polysorbate 80 | 210 |
| Maltodextrin | 56 |
| Stearic Acid | 28 |
| Aeroperl 300 | 140 |
| Hard gelatin capsule | 120 |
| Total weight with capsule shell | 668 |
| **Equal to 87,8 mg of net curcumin content* | |
| ***The total weight of the composition of the present invention within a single unit dose is 548 mg. Including the curcuminoid, emulsifier (Tween80), Dextrin compound, silica derivative and the acid component (pH decreasing agent).* | |

### Manufacturing Process:

1. The final composition from manufacturing example 1 is sieved through a 500um sieve and filled into hard gelatin capsules at about 668mg per each capsule.

## Claims

1. An oral pharmaceutical composition comprising at least one curcuminoid compound, at least one emulsifier, at least one silica, at least one dextrin compound and at least one acid component.

2. An oral composition according to claim 1, wherein the acid component is an organic acid.

3. An oral composition according to claim 1 or 2, wherein the acid component is selected from the group consisting of stearic acid, citric acid, gluconic acid (E574), inosinic acid (E630), glutamic acid (E620), guanilic acid (E626), sodium caprate (decanoic acid), dichroic acid (E330), malic acid (E296), acetic acid (E260), tartaric acid (E334), lactic acid (E270) and alginic acid (E400).

4. An oral composition according to claim 3, wherein the acid component is stearic acid.

5. An oral composition according to any one of the preceding claims, wherein the weight ratio of the curcuminoid compound to the dextrin compound is between 0.1:1 and 10:1.

6. An oral composition according to claim 5, wherein the weight ratio of the curcuminoid compound to the dextrin compound is between 0.5:1 and 8:1.

7. An oral composition according to any one of the preceding claims, wherein the weight ratio of the emulsifier to the acid component is between 40:1 and 1:1.

8. An oral composition according to claim 7, wherein the weight ratio of emulsifier to the acid component is between 30:1 and 2:1.

9. An oral composition according to any one of the preceding claims, wherein the weight ratio of the dextrin compound to the emulsifier is between 1:0.5 and 1:15.

10. An oral composition according to any one of the preceding claims, wherein the weight ratio of the silica to curcuminoid compound is between 0.5:1 and 10:1.

11. An oral composition according to any one of the preceding claims, wherein the curcuminoid compound is selected from the group consisting of demethoxycurcumin (DMC), bisdemethoxycurcumin (BDMC), tetrahydrocurcumin (THC) and cyclocurcumin.

12. An oral composition according to claim 9, wherein the curcuminoid compound is curcumin.

13. An oral composition according to any one of the preceding claims, wherein the emulsifier is selected from the group consisting of polyoxethylene, sorbitan esters, and polyethylene glycol.

14. An oral composition according to any one of the preceding claims, wherein the emulsifier is selected from the group consisting of stearoyl polyoxyl-32 glycerides, lauroyl polyoxyl-32 glycerides, polyoxyethylene sucrose diester dimyristate, polyoxyethylene sucrose diester dipalmitate, polyoxyethylene sucrose diester dioleate, polysorbate 80, polysorbate 60, polysorbate 20, PEG-8 laurate, PEG 400 monoluarate, PEG 10 isooctylphenyl ether, PEG 40 stearate, PEG 50 stearate, and PEG 40 isooctylphenyl ether.

15. An oral composition according to any one of the preceding claims, wherein the emulsifier is a polyoxylglyceride or a polysorbate (sorbitan ester).

16. An oral composition according to any one of the preceding claims, wherein the emulsifier is selected from the group consisting of stearoyl polyoxyl-32 glycerides, lauroyl polyoxyl-32 glycerides, polysorbate 80, polysorbate 60 and polysorbate 20.

17. An oral composition according to any one of the preceding claims, wherein the dextrin compound is selected from the group consisting of maltodextrin, α-cyclodextrin, γ-cyclodextrin, β-cyclodextrin, 2-hydroxypropyl-β-cyclodextrin, sulfobutylether β-cyclodextrin sodium salt, randomly methylated β-cyclodextrin, branched β-cyclodextrin and γ-cyclodextrin.

18. An oral composition according to claim 15, wherein the dextrin compound is maltodextrin or β-cyclodextrin.

19. An oral composition according to any one of the preceding claims, wherein the silica is a hydrophilic silica derivative.

20. An oral composition according to any one of the preceding claims, wherein the silica has a mean particle diameter of between 10 and 250 microns.

21. An oral composition according to any one of the preceding claims, wherein the silica has a BET surface area of between 40 and 400 m²/g.

22. An oral composition according to any one of the preceding claims, wherein the silica has a tamped density of between 50 and 600 g/L.

23. An oral composition according to claim 20, wherein the silica has a tamped density of between 50 and 400 g/L.

24. An oral composition according to any one of the preceding claims, wherein the silica is colloidal silicon dioxide, calcium silicate, or magnesium aluminometasilicate.

## Patentansprüche

1. Orale pharmazeutische Zusammensetzung, umfassend wenigstens eine Curcuminoidverbindung, wenigstens einen Emulgator, wenigstens eine Kieselerde, wenigstens eine Dextrinverbindung und wenigstens eine Säurekomponente.

2. Orale Zusammensetzung nach Anspruch 1, wobei die Säurekomponente eine organische Säure ist.

3. Orale Zusammensetzung nach Anspruch 1 oder 2, wobei die Säurekomponente ausgewählt ist aus der Gruppe bestehend aus Stearinsäure, Zitronensäure, Gluconsäure (E574), Inosinsäure (E630), Glutaminsäure (E620), Guanylsäure (E626), Natriumcaprat (Decansäure), dichroic acid (E330), Apfelsäure (E296), Essigsäure (E260), Weinsäure (E334), Milchsäure (E270) und Alginsäure (E400).

4. Orale Zusammensetzung nach Anspruch 3, wobei die Säurekomponente Stearinsäure ist.

5. Orale Zusammensetzung nach irgendeinem der vorhergehenden Ansprüche, wobei das Gewichtsverhältnis der Curcuminoidverbindung zu der Dextrinverbindung zwischen 0,1:1 und 10:1 liegt.

6. Orale Zusammensetzung nach Anspruch 5, wobei das Gewichtsverhältnis der Curcuminoidverbindung zu der Dextrinverbindung zwischen 0,5:1 und 8:1 liegt.

7. Orale Zusammensetzung nach irgendeinem der vorhergehenden Ansprüche, wobei das Gewichtsverhältnis von dem Emulgator zu der Säurekomponente zwischen 40:1 und 1:1 liegt.

8. Orale Zusammensetzung nach Anspruch 7, wobei das Gewichtsverhältnis von dem Emulgator zu der Säurekomponente zwischen 30:1 und 2:1 liegt.

9. Orale Zusammensetzung nach irgendeinem der vorhergehenden Ansprüche, wobei das Gewichtsverhältnis von der Dextrinverbindung zu dem Emulgator zwischen 1:0,5 und 1:15 liegt.

10. Orale Zusammensetzung nach irgendeinem der vorhergehenden Ansprüche, wobei das Gewichtsverhältnis von der Kieselerde zu der Curcuminoidverbindung zwischen 0,5:1 und 10:1 liegt.

11. Orale Zusammensetzung nach irgendeinem der vorhergehenden Ansprüche, wobei die Curcuminoidverbindung ausgewählt ist aus der Gruppe bestehend aus Desmethoxycurcumin (DMC), Bis-Desmethoxycurcumin (BDMC), Tetrahydrocurcumin (THC) und Cyclocurcumin.

12. Orale Zusammensetzung nach Anspruch 9, wobei die Curcuminoidverbindung Curcumin ist.

13. Orale Zusammensetzung nach irgendeinem der vorhergehenden Ansprüche, wobei der Emulgator ausgewählt ist aus der Gruppe bestehend aus Polyoxyethylen, Sorbitanestern und Polyethylenglykol.

14. Orale Zusammensetzung nach irgendeinem der vorhergehenden Ansprüche, wobei der Emulgator ausgewählt ist aus der Gruppe bestehend aus Stearoylpolyoxyl-32-Glyceriden, Lauroylpolyoxyl-32-Glyceriden, Polyoxyethylensucrosediesterdimyristat,Polyoxyethylensucrosediesterdip almitat, Polyoxyethylensucrosediesterdioleat, Polysorbat 80, Polysorbat 60, Polysorbat 20, PEG-8-Laurat, PEG-400-Monolaurat, PEG-10-Isooctylphenylether, PEG-40-Stearat, PEG-50-Stearat und PEG-40-Isooctylphenylether.

15. Orale Zusammensetzung nach irgendeinem der vorhergehenden Ansprüche, wobei der Emulgator ein Polyoxylglycerid oder ein Polysorbat (Sorbitanester) ist.

16. Orale Zusammensetzung nach irgendeinem der vorhergehenden Ansprüche, wobei der Emulgator ausgewählt ist aus der Gruppe bestehend aus Stearoylpolyoxyl-32-Glyceriden, Lauroylpolyoxyl-32-Glyceriden, Polysorbat 80, Polysorbat 60 und Polysorbat 20.

17. Orale Zusammensetzung nach irgendeinem der vorhergehenden Ansprüche, wobei die Dextrinverbindung ausgewählt ist aus der Gruppe bestehend aus Maltodextrin, α-Cyclodextrin, γ-Cyclodextrin, β-Cyclodextrin, 2-Hydroxypropyl-β-Cyclodextrin, Sulfobutylether-β-Cyclodextrin-Natriumsalz, zufällig methyliertem β-Cyclodextrin, verzweigtem β-Cyclodextrin und γ-Cyclodextrin.

18. Orale Zusammensetzung nach Anspruch 15, wobei die Dextrinverbindung Maltodextrin oder β-Cyclodextrin ist.

19. Orale Zusammensetzung nach irgendeinem der vorhergehenden Ansprüche, wobei die Kieselerde ein hydrophiles Kieselerdederivat ist.

20. Orale Zusammensetzung nach irgendeinem der vorhergehenden Ansprüche, wobei die Kieselerde einen mittleren Partikeldurchmesser zwischen 10 und 250 µm aufweist.

21. Orale Zusammensetzung nach irgendeinem der vorhergehenden Ansprüche, wobei die Kieselerde eine BET-Oberfläche zwischen 40 und 400 m²/g aufweist.

22. Orale Zusammensetzung nach irgendeinem der vorhergehenden Ansprüche, wobei die Kieselerde eine Stampfdichte zwischen 50 und 600 g/l aufweist.

23. Orale Zusammensetzung nach Anspruch 20, wobei die Kieselerde eine Stampfdichte zwischen 50 und 400 g/l aufweist.

24. Orale Zusammensetzung nach irgendeinem der vorhergehenden Ansprüche, wobei die Kieselerde kolloidales Siliciumdioxid, Calciumsilicat oder Magnesium-Aluminometasilicat ist.

## Revendications

1. Composition pharmaceutique orale comprenant au moins un composé de type curcuminoïde, au moins un émulsifiant, au moins une silice, au moins un composé de dextrine et au moins un composant de type acide.

2. Composition orale selon la revendication 1, dans laquelle le composant de type acide est un acide organique.

3. Composition orale selon la revendication 1 ou 2, dans laquelle le composant de type acide est choisi dans le groupe constitué par l'acide stéarique, l'acide citrique, l'acide gluconique (E574), l'acide inosinique (E630), l'acide glutamique (E620), l'acide guanilique (E626), le caprate de sodium (acide décanoïque), l'acide dichroïque (E330), l'acide malique (E296), l'acide acétique (E260), l'acide tartrique (E334), l'acide lactique (E270) et l'acide alginique (E400).

4. Composition orale selon la revendication 3, dans laquelle le composant de type acide est l'acide stéarique.

5. Composition orale selon l'une quelconque des revendications précédentes, dans laquelle le rapport en poids du composé de type curcuminoïde sur le composé de dextrine est compris entre 0.1:1 et 10:1.

6. Composition orale selon la revendication 5, dans laquelle le rapport en poids du composé de type curcuminoïde sur le composé de dextrine est compris entre 0.5:1 et 8:1.

7. Composition orale selon l'une quelconque des revendications précédentes, dans laquelle le rapport en poids de l'émulsifiant sur le composant de type acide est compris entre 40:1 et 1:1.

8. Composition orale selon la revendication 7, dans laquelle le rapport en poids d'émulsifiant sur le composant de type acide est compris entre 30:1 et 2:1.

9. Composition orale selon l'une quelconque des revendications précédentes, dans laquelle le rapport en poids du composé de dextrine sur l'émulsifiant est compris entre 1:0.5 et 1:15.

10. Composition orale selon l'une quelconque des revendications précédentes, dans laquelle le rapport en poids de la silice sur le composé de type curcuminoïde étant compris entre 0.5:1 et 10:1.

11. Composition orale selon l'une quelconque des revendications précédentes, dans laquelle le composé de type curcuminoïde est choisi dans le groupe constitué par la déméthoxycurcumine (DMC), la bisdéméthoxycurcumine (BDMC), la tétrahydrocurcumine (THC) et la cyclocurcumine.

12. Composition orale selon la revendication 9, dans laquelle le composé de type curcuminoïde est la curcumine.

13. Composition orale selon l'une quelconque des revendications précédentes, dans laquelle l'émulsifiant est choisi dans le groupe constitué par un polyoxyéthylène, des esters de sorbitane et un polyéthylène glycol.

14. Composition orale selon l'une quelconque des revendications précédentes, dans laquelle l'émulsifiant est choisi dans le groupe constitué par des stéaroyl-polyoxyl-32 glycérides, des lauroyl-polyoxyl-32 glycérides, un dimyristate de diester de polyoxyéthylène saccharose, un dipalmitate de diester de polyoxyéthylène saccharose, un dioléate de diester de polyoxyéthylène saccharose, le polysorbate 80, le polysorbate 60, le polysorbate 20, le laurate de PEG-8, le monolaurate de PEG 400, l'éther isooctylphénylique de PEG 10, le stéarate de PEG 40, le stéarate de PEG 50 et l'éther isooctylphénylique de PEG 40.

15. Composition orale selon l'une quelconque des revendications précédentes, dans laquelle l'émulsifiant est un polyoxylglycéride ou un polysorbate (ester de sorbitane).

16. Composition orale selon l'une quelconque des revendications précédentes, dans laquelle l'émulsifiant est choisi dans le groupe constitué par des stéaroyl-polyoxyl-32 glycérides, des lauroyl-polyoxyl-32 glycérides, le polysorbate 80, le polysorbate 60 et le polysorbate 20.

17. Composition orale selon l'une quelconque des revendications précédentes, dans laquelle le composé de dextrine est choisi dans le groupe constitué par une maltodextrine, une α-cyclodextrine, une γ-cyclodextrine, une β-cyclodextrine, une 2-hydroxypropyl-β-cyclodextrine, un sel de sodium de sulfobutyléther de β-cyclodextrine, une β-cyclodextrine méthylée de manière aléatoire, une β-cyclodextrine ramifiée et une γ-cyclodextrine.

18. Composition orale selon la revendication 15, dans laquelle le composé de dextrine est une maltodextrine ou une β-cyclodextrine.

19. Composition orale selon l'une quelconque des revendications précédentes, dans laquelle la silice est un dérivé de silice hydrophile.

20. Composition orale selon l'une quelconque des revendications précédentes, dans laquelle la silice a un diamètre moyen de particule compris entre 10 et 250 microns.

21. Composition orale selon l'une quelconque des revendications précédentes, dans laquelle la silice a une superficie BET comprise entre 40 et 400 m²/g.

22. Composition orale selon l'une quelconque des revendications précédentes, dans laquelle la silice a une densité tassée comprise entre 50 et 600 g/L.

23. Composition orale selon la revendication 20, dans laquelle la silice a une densité tassée comprise entre 50 et 400 g/L.

24. Composition orale selon l'une quelconque des revendications précédentes, dans laquelle la silice est un dioxyde de silicium colloïdal, un silicate de calcium ou un aluminométasilicate de magnésium.
